(19) Europäisches Patentamt
European Patent Office
Office européen des brevets

(11) Veröffentlichungsnummer: **0 008 099**
**B1**

(12) **EUROPÄISCHE PATENTSCHRIFT**

(21) Anmeldenummer: **79102808.7**

(22) Anmeldetag: **04.08.79**

(51) Int. Cl.³: **C 07 C 69/16, C 07 C 67/05**

(54) **Verfahren zur Herstellung von Carbonsäureestern vicinaler Glykole.**

(30) Priorität: **07.08.78 DE 2834540**

(43) Veröffentlichungstag der Anmeldung:
**20.02.80 Patentblatt 80/4**

(45) Bekanntmachung des Hinweises auf die Patenterteilung:
**25.03.81 Patentblatt 81/12**

(84) Benannte Vertragsstaaten:
**BE DE FR GB IT NL**

(56) Entgegenhaltungen:
**FR - A - 2 092 924**
**FR - A - 2 352 782**

**Chemical Engineers' Handbook R. H. Perry/C.H. Chilton, Abteilung 26, Seite 60**

**Handbook of Chemistry and Physics, 59th Edition, Seiten B 65, B 66**

(73) Patentinhaber: **BASF Aktiengesellschaft**
**Carl-Bosch-Strasse 38**
**D-6700 Ludwigshafen (DE)**

(72) Erfinder: **Platz, Rolf, Dr.**
**Hansastrasse 5**
**D-6800 Mannheim 1 (DE)**

(72) Erfinder: **Weitz, Hans-Martin, Dr.**
**Auf dem Koeppel 40**
**D-6702 Bad Duerkheim 1 (DE)**

(72) Erfinder: **Hartig, Juergen, Dr.**
**In der Schleit 9**
**D-6718 Gruenstadt (DE)**

**EP 0 008 099 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Verfahren zur Herstellung von Carbonsäureestern vicinaler Glykole

Die vorliegende Erfindung betrifft ein verbessertes Verfahren zur Herstellung von Carbonsäureestern vicinaler Glykole durch katalytische Oxidation von Olefinen mit Sauerstoff und in Gegenwart von Carbonsäuren in der Flüssigphase.

Aus der US—PS 3 985 795 und der DE—OS 26 32 158 ist es bekannt, Olefine in der Flüssigphase in Gegenwart von Halogenen und Tellursalzen zu Carbonsäure- Mono- und Diestern der dem Olefin entsprechenden vicinalen Glykole umzusetzen.

Besondere Bedeutung hat hierbei die Umsetzung von Äthylen oder Propylen mit Essigsäure in Gegenwart von Halogenen oder Halogen abspaltenden Verbindungen sowie in Gegenwart von Tellurkationen bei erhöhter Temperatur und unter erhöhtem Druck gemäß folgender Gleichung

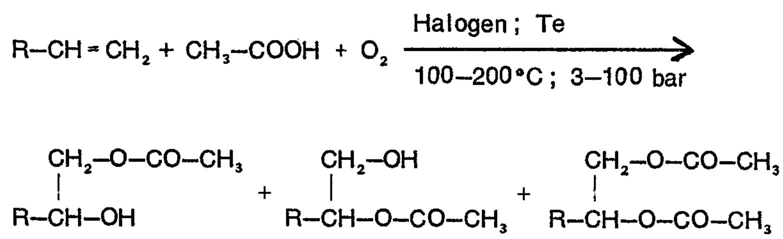


$R = H;\ CH_3$

da diese Ester unter Abspaltung von Essigsäure in Äthylenoxid bzw. Propylenoxid überführt werden können.

Obwohl diese Reaktion mit hoher Raum-Zeit-Ausbeute verläuft und für sich betrachtet einen sehr vorteilhaften Teilschritt auf dem Weg zur Herstellung von Alkylenoxiden darstellt, hat sie bisher keinen Eingang in die Technik gefunden. Das saure, Sauerstoff, Halogen und die Tellursalze enthaltende Reaktionsgemisch ist unter den Verfahrensbedingungen derart aggressiv, daß bisher noch kein korrosionsfester Werkstoff gefunden wurde, der eine wirtschaftliche Verwertung dieses Oxidationsverfahrens gestattet hätte.

Normalerweise auch unter recht energischen Bedingungen korrosionsfeste Stähle wie Chrom-Nickel-Molybdän-Stähle zeigten bereits nach wenigen Betriebsstunden eine nicht mehr tolerierbare Korrosion und selbst Titan war nicht brauchbar. Lediglich Tantal erwies sich bisher als korrosionsbeständig, aber da dieser Werkstoff extrem teuer ist, kommt er aus wirtschaftlichen Gründen für großtechnische Anlagen praktisch nicht in Betracht.

Der Erfindung lag daher die Aufgabe zugrunde, einen Werkstoff für Reaktionsgefäße und Apparaturen zu finden, in denen sich das genannte Verfahren ohne Korrosionsschwierigkeiten ausführen läßt.

Es wurde gefunden, daß man Carbonsäureester vicinaler Glykole durch Oxidation von Olefinen mit Sauerstoff und in Gegenwart von Carbonsäuren sowie von Halogenen und Tellursalzen als Katalysatoren ohne verfahrenstechnische, durch Korrosionen bedingte Schwierigkeiten in der Flüssigphase erhält, wenn man diese Reaktion in Gefäßen und Apparaturen ausführt, die aus Zirkon oder Legierungen aus mindestens 80 Gew.% Zirkon und dem Rest Zinn und/oder Hafnium bestehen oder die mit diesen Materialien ausgekleidet sind.

Die erfindungsgemäße Verwendung von Zirkon bzw. der Zirkonlegierungen empfiehlt sich allgemein für solche definitionsgemäßen Reaktionsgemische, die neben den nicht korrosiven Einsatzstoffen, den Olefinen, und neben den Verfahrensprodukten, den Estern, folgende Mengen der korrosiven Substanzen enthalten:

| | |
|---|---|
| 40—95 Gew.% | einer Carbonsäure |
| 0,5—10 Gew.% | Halogen wie Chlor, Brom und/oder Jod in elementarer und/oder ionischer Form |
| 0,05—5 Gew.% | Tellurdioxid oder der äquimolaren Menge eines Tellursalzes, z.B. eines Halogenides, und |
| 0,002—0,3 Gew.% | Sauerstoff, entsprechend einem Sauerstoffpartialdruck von etwa 0,5—20 bar. |

Handelt es sich bei der Oxidationsreaktion um die eingangs erwähnte, technisch besonders bedeutsame Herstellung der Äthylenglykol- und Propylenglykolacetate, so besteht ein derartiges Reaktionsgemisch aus

| | |
|---|---|
| 40—90 Gew.% | Essigsäure |
| 0,5—10 Gew.% | Halogen, insbesondere Jod, |
| 0,05—5 Gew.% | Tellurdioxid |
| 0,002—0,3 Gew.% | Sauerstoff, entsprechend einem Sauerstoffpartialdruck von 0,5—20 bar |
| 1—5 Gew.% | Wasser |
| 3—20 Gew.% | des Olefins und |
| 2—55 Gew.% | des Estergemisches als Verfahrensprodukt. |

Bis zu einer Temperatur von 180°C und einem Druck von 80 bar wurden keine nennenswerten Korrosionserscheinungen beobachtet, so daß auch unter noch schärferen Bedingungen keine sprunghafte Zunahme der Korrosion zu erwarten ist. Im allgemeinen dürfte es aber keine Vorteile mehr bringen, die Oxidationsreaktion oberhalb von 180°C und 80 bar auszuführen.

Im Vergleich zu herkömmlichen Materialien wie Chrom-Nickel-Molybdänstählen oder Titan, bieten Zirkon oder die definitionsgemäßen Zirkonlegierungen bereits bei 110°C und 3 bar, den unteren Grenzwerten, bei denen die Oxidationsreaktionen technisch ausgeführt werden, deutliche Vorteile.

Zirkon und seine Legierungen sind an sich bekannte Werkstoffe und können nach den hierfür bekannten Verfahren zu den Reaktionsgefäßen und Apparaturen aller Art verarbeitet werden. Das gleiche gilt für Auskleidungen, also Verbundwerkstoffe wie Zirkon auf Stahl. Normalerweise enthält handelsübliches Zirkon bereits etwa 5 Gew.% seines natürlichen Begleiters Hafnium, und nur für Spezialzwecke wird auch das Hafnium gänzlich oder zum Teil entfernt. Für den vorliegenden Zweck ist das teurere reine Zirkon zwar selbstverständlich geeignet, bietet jedoch gegenüber dem Hafnium enthaltenden Zirkon keine Vorteile.

Die gute Eignung von Zirkon bzw. seinen Legierungen für den erfindungsgemäßen Zweck verdient im Hinblick auf die Unbrauchbarkeit des in seinen chemischen Eigenschaften sehr ähnlichen Titans als besonders bemerkenswert hervorgehoben zu werden. Als ebenso korrosionsfest ist bisher nur Tantal bekannt geworden. Die Preise für Apparate aus Tantal sind aber etwa dreimal höher, als die für Apparate aus Zirkon.

## Beispiel

Materialproben (mit je einer Schweißnaht) aus

A einem handelsüblichen Zirkonblech mit einem Hafnium-Gehalt von 4,5 Gew.%
B einem handelsüblichen Blech einer Legierung aus rund 94 Gew.% Zirkon, 4 Gew.% Hafnium und 2 Gew.% Zinn und, im Vergleich dazu
C einem handelsüblichen Tantalblech sowie
D einem handelsüblichen Titanblech

wurden gemäß einem üblichen Korrosionstest in einem Modell-Reaktionsgemisch aus

| | |
|---|---|
| 1 000 g | Essigsäure |
| 200 g | eines Gemisches aus Propandiolmonoacetaten und -diacetat |
| 15 g | Wasser |
| 25 g | Jod und |
| 7 g | Tellurdioxid |

unter Sauerstoffatmosphäre 30 Tage lang unter Rühren auf a) 118°C und, parallel dazu, b) auf 180°C erhitzt.

Bei 118°C betrug der Druck 1 bar, entsprechend einem Sauerstoffgehalt von rund 1 g in dem Reaktionsgemisch, und bei 180°C betrug der Druck 25 bar, entsprechend einem $O_2$-Partialdruck von etwa 20 bar oder einem Gehalt von rund 16 g $O_2$ in dem Gemisch.

Die hierbei festgestellten Materialverluste wurden auf eine Korrosionsgeschwindigkeit von mm/Jahr umgerechnet.

Die Ergebnisse sind der folgenden Tabelle zu entnehmen.

| Material | Korrosion in mm/Jahr bei 118°C | 180°C |
|---|---|---|
| A Zirkon/(Hf) | 0,004 | 0,004 |
| B Zr/(Hf)/Sn | 0,018 | 0,036 |
| C Tantal | 0,001 | 0,001 |
| D Titan | 0,24 +) | 10,7 +) |

+) Lochfraß, besonders an den Schweißnähten.

Die Ergebnisse sprechen für sich selbst, wobei zu berücksichtigen ist, daß Werkstoffe mit Flächenkorrosionen mit Werten bis 0,1 mm/Jahr als "gut geeignet" gelten.

**Patentanspruch**

Verfahren zur Herstellung von Carbonsäureestern vicinaler Glykole durch Oxidation von Olefinen mit Sauerstoff und in Gegenwart von Carbonsäuren sowie von Halogen und von Tellursalzen als Katalysatoren, dadurch gekennzeichnet, daß man diese Reaktion in Gefäßen und Apparaten ausführt, die aus Zirkon oder Legierungen aus mindestens 80 Gew.% Zirkon und dem Rest Zinn und/oder Hafnium bestehen oder die mit diesen Materialien ausgekleidet sind.

**Revendication**

Procédé de préparation d'esters d'acides carboxyliques de glycols vicinaux par oxydation d'oléfines par l'oxygène en présence d'acides carboxyliques, ainsi que d'halogènes et de sels du tellure comme catalyseurs, caractérisé en ce que cette réaction est effectuée dans un appareillage et des réacteurs réalisés en zirconium ou en un alliage composé à raison d'au moins 80% en poids de zirconium, le restant étant de l'étain et(ou) du hafnium, ou pourvus d'un revêtement de zirconium ou d'un tel alliage.

**Claim**

A process for the preparation of carboxylic acid esters of vicinal glycols by oxidizing olefins with oxygen in the presence of carboxylic acids and of halogen and tellurium salts as catalysts, characterized in that this reaction is carried out in vessels and apparatus consisting of, or lined with, zirconium or alloys of not less than 80% by weight of zirconium, the remainder being tin and/or hafnium.